Europäisches Patentamt

European Patent Office

Office européen des brevets

(11) Publication number: **0 174 123**
**A2**

(12) **EUROPEAN PATENT APPLICATION**

(21) Application number: **85305831.1**

(22) Date of filing: **16.08.85**

(51) Int. Cl.$^4$: **C 07 D 307/36**

(30) Priority: **01.09.84 GB 8422162**

(43) Date of publication of application:
**12.03.86 Bulletin 86/11**

(84) Designated Contracting States:
**AT BE CH DE FR GB IT LI LU NL SE**

(71) Applicant: **The British Petroleum Company p.l.c.**
**Britannic House Moor Lane**
**London EC2Y 9BU(GB)**

(72) Inventor: **Watson, William John West**
**The British Petroleum Company p.l.c. Chertsey Road**
**Sunbury-on-Thames Middlesex, TW16 7LN(GB)**

(74) Representative: **Eastman, Hugh Leonard et al,**
**BP INTERNATIONAL LIMITED Patents Division Chertsey**
**Road**
**Sunbury-on-Thames Middlesex, TW16 7LN(GB)**

(54) **Process for the preparation of alkylfurans.**

(57) A process for the preparation of alkylfurans comprising reacting furan with an alkene in the presence of a supported phosphoric acid catalyst wherein the alkene has from 4 to 20 carbon atoms and is a branched chain alkene which is a 1,1-disubstituted ethene or a tri- or tetra-substituted ethene and the molar ratio of furan to alkene is at least 1:1. The catalyst is supported on a silica-containing support which is preferably manufactured silica or a naturally occurring silicaceous composition e.g. kieselguhr. The alkylfurans may be used as a gasoline additive.

EP 0 174 123 A2

## PROCESS FOR THE PREPARATION OF ALKYLFURANS

The present invention relates to a process for the preparation of alkylfurans.

It is known to be difficult to alkylate furans using the Friedel-Crafts reaction. Such reactions give low yields of alkylfurans due to resinification which consumes furan and deactivates the Friedel-Crafts catalyst by encapsulation.

Shuikin et al, Doklady Akademii Nauk SSSR 173(5) 621-623 (1967), Izvestiya Akademii Nauk SSSR Serija Khimicheskaya 1967(7), 1618-1620 and Izvestiya Akademii Nauk SSR 1968(11), 2618-2620, disclose processes capable of producing alkylated furans in relatively high yields. The processes comprise reacting furan with an alkene in the presence of a catalyst which comprises phosphoric acid on kieselguhr. The reactions were carried out in an autoclave at temperatures from 50 to 175°C using an excess of furan over the alkene.

It has now been found that the use of at least a stoichiometric amount of alkene to furan and preferably an excess of alkene over the furan unexpectedly reduces the amount of resin produced and may increase the yield of alkyl furans.

Thus, according to the present invention a process for the preparation of alkylfurans comprises reacting furan with an alkene in the presence of a supported phosphoric acid catalyst characterised in that the alkene has from 4 to 20 carbon atoms and is a branched chain alkene which is a 1,1 disubstituted ethene or a tri- or tetra-substituted ethene and in that the molar ratio of

1

furan to alkene is at least 1:1.

Furan may be used alone or in admixture with a minor proportion of other components such as, for example, substituted furans.

The alkene is a branched chain alkene which is a 1,1 disubstituted ethene or a tri- or tetra-substituted ethene (i.e. an alkene capable of forming a tertiary carbonium ion on protonation). The alkene preferably has from 4 to 6 carbon atoms. Preferred alkenes include 2-methyl-but-1-ene and2-methyl-but-2-ene. Mixtures of alkenes may be used.

The molar ratio of furan to branched chain alkene is preferably from 1.0:1.0 to 1.0: 6.0 and is more preferably from 1.0:1.01 to 1.0:2.0.

The catalyst comprises phosphoric acid on a suitable support which may be any of the supports known for use with phosphoric acid. Preferably the phosphoric acid is supported on manufactured silica or on a naturally occuring silicaceous composition e.g. kieselguhr. At similar loadings of phosphoric acid, an $H_3PO_4$/kieselguhr catalyst is more active than an $H_3PO_4$/silica catalyst but is less selective. The amount of catalyst used will depend on the amount of phosphoric acid on the support. Typically the support carries 25 to 35% wt of phosphoric acid. Increasing the amount of phosphoric acid used in the reaction tends to give a higher yield of alkylfurans but also tends to increase the yield of solid resinous products. Preferably the amount of phosphoric acid used is from $10^{-4}$ to $3 \times 10^{-2}$ moles per mole of furan, more preferably from $5 \times 10^{-3}$ to $2 \times 10^{-2}$ moles per mole.

The reaction temperature may be from 20 to 300°C but is preferably from 50 to 200°C. A closed vessel must be used to contain the furan and the alkene but high pressures are not essential. The reaction pressure may, for example, be from $1.01 \times 10^2$ kN m$^{-2}$ to $1.01 \times 10^4$ kN m$^{-2}$ and is preferably from $3.04 \times 10^2$ kN m$^{-2}$ to $5.06 \times 10^3$ kN m$^{-2}$. Depending upon the temperature and pressure, the reaction time may be from 1 to 24 hours. Preferably the reaction time is from 1 to 24 hours, more preferably from 2 to 10 hours.

It is known that alkylfurans may be included in gasoline compositions as octane improving additives and the product of the process according to the present invention is particularly useful for alkylating furan for this use. Since an excess of alkene is used in the process according to the invention, the product contains relatively small amounts of unreacted furan which is advantageous when the product is to be used as a gasoline additive.

The invention is illustrated by the following examples.

Example 1

15.6 ml of orthophosphoric acid (specific gravity 1.75, 85% $H_3PO_4$) was added to 134.4 ml of distilled water. This solution was then added to 50g of kieselguhr (41.8% wt Si, 1.58% wt Al, 1.69% wt Na) and the mixture agitated for seven hours. The mixture was allowed to stand for 16 hours at room temperature (approximately 20°C), the bulk water was then removed by evaporation under reduced pressure and the product was dried at 120°C for 24 hours under a reduced pressure of approximately 50 kN $m^{-2}$. The $H_3PO_4$/kieselguhr catalyst produced contained 9.8% wt of phosphorous which is equivalent to 31% wt of phosphoric acid.

2g of the $H_3PO_4$/kieselguhr catalysts were placed in a 200 ml capacity autoclave together with 38.3g of furan and 40.0g of 2-methyl-but-1-ene. The autoclave was heated to 100°C over a period of 15 minutes and the temperature maintained at 100°C for 3 hours. The autoclave was then allowed to cool to room temperature (approximately 20°C). The catalyst was removed by filtration and the liquid product analysed by gas-liquid chromatography.

Details of the product yield are given in Table 1.

Example 2

Example 1 was repeated except that an $H_3PO_4$/silica catalyst was used in place of the $H_3PO_4$/kieselguhr catalyst. The catalyst was prepared as described in Example 1 except that 50g of silica gel (41.8% wt Si, 0.06% wt Na) was used in place of the kieselguhr.

Details of the product yield are given in Table 1.

Comparative Examples A and B

In Examples 1 and 2 a slight excess (0.014 mole) of 2-methyl-but-1-ene was used over the furan.. For comparison, Examples 1 and 2 were repeated except that an excess of furan was used i.e. 65.7g of furan and 22.1g of 2-methyl-but-1-ene.

In Examples 1 and 2 according to the invention substantially all of the product was liquid whereas in the comparative examples A and B relatively large amounts of resin were produced as indicated by a liquid recovery of only 74 and 75% by weight respectively compared with a total recovery of 89 and 85% by weight respectively.

Examples 3 to 5

Example 1 was repeated except that 2-methyl-but-2-ene was used instead of 2-methyl-but-1-ene. In Example 4, the duration of the heating at 100°C was increased to 4 hours and in Example 5, 3g of the $H_3PO_4$/kieselguhr catalyst was used.

Details of the product yield are given in Table 2.

Example 6

30.1g of furan and 45.7g of 2-methyl but-1-ene were reacted together in an autoclave in the presence of the $H_3PO_4$/silica catalyst used in Example 2. The reaction conditions were as for Examples 1 and 2.

Details of the product yield are given in Table 3.

Example 7

Example 6 was repeated except that the amounts of furan and 2-methyl-but-1-ene were 10.0g and 60.5g respectively.

Details of the product yield are given in Table 3.

Example 8

23.95g of furan and 51.65g of 2-methyl-but-2-ene were reacted together in the presence of the $H_3PO_4$/kielseguhr catalyst as prepared in Example 1. The reaction conditions were as for Example 1

Details of the product are given in Table 3.

Table 1

| Ex | Catalyst | Liquid Recovery (% wt) | Total Recovery (% wt) | Furan Conversion (%) | Selectivity based on furan conversion | Olefin Conversion (%) | Selectivity based on olefin conversion | Yield of Alkyl Furans (% of theoretical) |
|---|---|---|---|---|---|---|---|---|
| 1 | $H_3PO_4$/kieselguhr | 93 | 94 | 97 | 77 | 78 | 98 | 78 |
| 2 | $H_3PO_4$/silica | 91 | 92 | 87 | 77 | 73 | 94 | 74 |

Table 2

| Ex | Weight of catalyst (g) | Duration of Reaction (hrs) | Liquid Recovery (% wt) | Total Recovery (% wt) | Furan Conversion (%) | Selectivity based on Furan Conversion | Olefin Conversion (%) | Selectivity based on Olefin Conversion | Yield of Alkylfurans (% of theoretical) |
|---|---|---|---|---|---|---|---|---|---|
| 3 | 2 | 3 | 94 | 94 | 97 | 77 | 78 | 98 | 78 |
| 4 | 2 | 4 | 95 | 96 | 97 | 79 | 77 | 99 | 78 |
| 5 | 3 | 3 | 94 | 96 | 99 | 77 | 80 | 99 | 80 |

## Table 3

| Ex | Liquid Recovery (% wt) | Total Recovery (% wt) | Furan Conversion (%) | Selectivity based on Furan Conversion | Olefin Conversion (%) | Selectivity based on Olefin Conversion | Yield of Alkylfurans (% of theoretical) |
|---|---|---|---|---|---|---|---|
| 6 | 87 | 88 | 94 | 77 | 67 | 77 | 74 |
| 7 | 90 | 92 | 100 | 67 | 62 | 24 | 66 |
| 8 | 91 | 94 | 99 | 81 | 59 | 68 | 80 |

Claims:

1. A process for the preparation of alkyl furans comprises reacting furan with an alkene in the presence of a supported phosphoric acid catalyst characterised in that the alkene has from 4 to 20 carbon atoms and is a 1,1 disubstituted ethene or a tri- or tetra-substituted ethene and in that the molar ratio of furan to alkene is at least 1:1.

2. A process as claimed in claim 1 in which the molar ratio of furan to alkene is from 1:1 to 1:6.

3. A process as claimed in claim 2 in which the molar ratio of furan to alkene is from 1:1.01 to 1:2.

4. A process as claimed in any one of claims 1 to 3 in which the alkene has from 4 to 6 carbon atoms.

5. A process as claimed in claim 4 in which the alkene is 2-methyl-but-1-ene, 2-methyl-but-2-ene or a mixture thereof.

6. A process as claimed in any one of claims 1 to 5 in which the phosphoric acid catalyst comprises from 25 to 35% by weight of phosphoric acid supported on manufactured silica or a naturally occurring silicaceous composition.

7. A process as claimed in claim 6 in which the phosphoric acid is supported on kieselguhr.

8. A process as claimed in any one of claims 1 to 7 in which the amount of phosphoric acid used is from $5 \times 10^{-3}$ to $2 \times 10^{-2}$ moles per mole of furan.

9. A process as claimed in any one of claims 1 to 8 in which the reaction is carried out at a temperature of from 20 to 300°C for a period of from 1 to 24 hours.

10. A process as claimed in claim 9 in which the reaction is carried out at a temperature of from 50 to 200°C for a period of from 2 to 10 hours.